# FASCICULE DE BREVET EUROPEEN

(11) **EP 1 142 564 B1**
(45) Date de publication et mention de la délivrance du brevet: **15.06.2005**
(21) Numéro de dépôt: 01400253.9
(22) Date de dépôt: 01.02.2001
(51) Int. Cl.: A61K 7/48, A61K 7/42

(54) **Compositions photoprotectrices comprenant un mélange d'un polyéthylène à fonction acide carboxylique terminale et d'un polyéthylène**
Lichtschutzzubereitungen enthaltend ein Gemisch von Polyethylene mit einer carboxilischen Gruppe und Polyethylene
Sunscreen compositions comprising a mixture of polyethylene with a carboxylic function and polyethylene

(30) Priorité: 08.02.2000 FR 0001535
(43) Date de publication de la demande: 10.10.2001
(73) Titulaire: L'OREAL, 75008 Paris (FR)
(72) Inventeur: Josso, Martin, 75005 Paris (FR); Meurisse, Stéphanie, 95390 Saint Prix (FR)
(74) Mandataire: Andral, Christophe André Louis

(56) Documents cités:
- WO-A-93/04666
- WO-A-97/12584
- WO-A-97/42933
- WO-A-98/46200
- US-A- 5 208 011
- US-A- 5 250 289
- US-A- 5 417 961
- US-A- 5 770 183
- US-A- 6 007 797
- DATABASE WPI Section Ch, Week 198652 Derwent Publications Ltd., London, GB; Class D21, AN 1986-343347 XP002157184 & JP 61 257910 A (POLA KASEI KOGYO KK), 15 novembre 1986 (1986-11-15)
- DATABASE WPI Section Ch, Week 199851 Derwent Publications Ltd., London, GB; Class D21, AN 1998-603233 XP002157185 & JP 10 273433 A (SHISEIDO CO LTD), 13 octobre 1998 (1998-10-13)
- DATABASE WPI Section Ch, Week 199706 Derwent Publications Ltd., London, GB; Class D21, AN 1997-061716 XP002157186 & JP 08 310941 A (NOEVIR KK), 26 novembre 1996 (1996-11-26)
- "Formulary/March 2000" HAPPI MAGAZINE, [en ligne] XP002157182 Extrait de l'Internet: <URL:http://www.happi.com/special/0300form .htm> [extrait le 2001-01-11]
- "Personal Care Industry" MCCULLOUGH AND BENTON- PERSONAL CARE, [en ligne] XP002157183 Extrait de l'Internet: <URL:http://www.mcculloughandbenton.com/pe rsonalcare.htm> [extrait le 2001-01-11]

## Description

La présente invention concerne des compositions cosmétiques et/ou dermatologiques destinées à la protection de la peau et/ou des cheveux contre les rayonnements UV, en particulier le rayonnement solaire comprenant, dans un support cosmétiquement et/ou dermatologiquement acceptable, au moins un système photoprotecteur capable de filtrer le rayonnement UV et au moins un mélange de polyéthylènes constitué d'au moins un polyéthylène à fonction acide carboxylique terminale sous forme libre, partiellement ou totalement neutralisée et d'au moins un polyéthylène.

La présente invention concerne également l'utilisation d'un mélange de polyéthylènes constitué d'au moins un polyéthylène à fonction acide carboxylique terminale sous forme libre, partiellement ou totalement neutralisée et d'au moins un polyéthylène dans la préparation de compositions cosmétiques et/ou dermatologiques destinées à la protection de la peau et/ou des cheveux contre les rayonnements UV, en particulier le rayonnement solaire dans le but d'augmenter le facteur de protection solaire (SPF).

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV).

Or, à la suite d'importantes recherches menées dans le domaine de la photoprotection évoqué ci-dessus, la Demanderesse a maintenant découvert, de façon inattendue et surprenante, qu'en associant dans un support du type émulsion huile-dans-eau un mélange de polyéthylènes particulier que l'on définira plus loin à un système photoprotecteur, il était possible d'obtenir des compositions antisolaires ayant des indices de protection supérieurs à ceux qui peuvent être obtenus avec le même système photoprotecteur seul.

Cette découverte est à la base de la présente invention.

Ainsi, conformément à un premier objet de la présente invention, il est proposé de nouvelles compositions, plus particulièrement destinées à la protection de la peau et/ou des cheveux contre le rayonnement ultraviolet, caractérisées par le fait qu'elles comprennent, dans un support cosmétiquement acceptable du type émulsion huile-dans-eau, au moins :
(a) un système photoprotecteur capable de filtrer le rayonnement UV ;
(b) un mélange de polyéthylènes constitué d'au moins un polyéthylène à fonction acide carboxylique terminale et d'au moins un polyéthylène.

Selon l'invention, on entend désigner de manière générale par système photoprotecteur capable de filtrer le rayonnement UV, tout composé ou toute association de composés qui, par des mécanismes connus en soi d'absorption et/ou de réflexion et/ou diffusion du rayonnement UV-A et/ou UV-B, permet d'empêcher, ou du moins limiter, la mise en contact dudit rayonnement avec une surface (peau, cheveux,) sur laquelle ce ou ces composés ont été appliqués. En d'autres termes, ces composés peuvent être des filtres organiques photoprotecteurs absorbeurs d'UV ou des (nano)pigments minéraux diffuseurs et/ou réflecteurs d'UV, ainsi que leurs mélanges.

Un autre objet encore de la présente invention réside dans l'utilisation d'un mélange de polyéthylènes constitué d'au moins un polyéthylène à fonction acide carboxylique terminale et d'au moins un polyéthylène pour la fabrication d'une composition destinée à la protection de la peau et/ou des cheveux contre le rayonnement ultraviolet, comprenant dans un support du type émulsion huile dans eau un système photoprotecteur capable de filtrer le rayonnement UV, comme agent permettant d'augmenter le facteur de protection solaire (SPF).

D'autres caractéristiques, aspects et avantages de la présente invention apparaîtront à la lecture de la description détaillée qui va suivre.

Les mélanges de polyéthylène conformes à la présente invention comprennent en général de 60 à 99% en poids et plus préférentiellement de 80 à 90% en poids en polyéthylène à fonction acide carboxylique terminale et de 1 à 40% en poids et plus préférentiellement de 10 à 20% en poids de polyéthylène par rapport poids total du mélange.

Dans le mélange de polyéthylènes conformes à l'invention, la fonction acide carboxylique terminale peut être éventuellement partiellement ou totalement neutralisée par un base minérale telle que la soude, la potasse et l'ammoniaque ou bien une base organique telle qu'une amine ou une alcanolamine telle que la triéthanolamine.

Les mélanges de polyéthylène conformes à la présente invention ont un poids moléculaire moyen en nombre allant de 300 à 800, présentent un indice d'acide allant de 60 à 120 et un point de fusion allant de 80 à 120°C.

Parmi les mélanges de polyéthylène conformes à la présente invention , on utilisera plus particulièrement les produits vendus sous la dénomination commerciale «PERFORMACID» par la société NEW PHASE TECHNOLOGIES tels que par exemple :
- le mélange polyéthylène à fonction acide carboxylique terminale/polyéthylène de poids moléculaire 350, d'indice d'acide 115 et de point de fusion 89°C (nom INCI : C₂₀-C₄₀ ACID et POLYETHYLENE) vendu sous le nom commercial « PERFORMACID 350 » ;
- le mélange polyéthylène à fonction acide carboxylique terminale/polyéthylène de poids moléculaire 460, d'indice d'acide 94 et de point de fusion 94°C vendu sous le nom commercial « PERFORMACID 425 » ;
- le mélange polyéthylène à fonction acide carboxylique terminale/polyéthylène de poids moléculaire 550, d'indice d'acide 72,5 et de point de fusion 101°C vendu sous le nom commercial PERFORMACID 550 ;
- le mélange polyéthylène à fonction acide carboxylique terminale/polyéthylène de poids moléculaire 700, d'indice d'acide 63 et de point de fusion 110°C vendu sous le nom commercial PERFORMACID 700.

Les mélanges de polyéthylènes conformes à la présente invention sont utilisés dans les compositions conformes à l'invention à des concentrations allant de préférence de 0,1 à 10% en poids par rapport au poids total de la composition et plus particulièrement de 0,2 à 5% en poids.

Le système photoprotecteur selon l'invention est généralement présent dans les compositions selon l'invention à une teneur allant de 0,1 % à 30 % en poids et de préférence de 0,5 à 15 %, en poids, par rapport au poids total de la composition.

Selon l'invention, le système photoprotecteur peut être constitué par un ou plusieurs filtres organiques hydrophiles et/ou un ou plusieurs filtres organiques lipophiles et/ou un ou plusieurs (nano)pigments minéraux.

Les filtres organiques, actifs dans l'UV-A et/ou l'UV-B, hydrophiles ou lipophiles peuvent être choisis notamment parmi les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone ; les dérivés de β,β'-diphénylacrylate, les dérivés de benzimidazole ; les dérivés bis-benzoazolyle tels que ceux décrits dans les brevets EP-A-0669323 et US 2,463,264 ; les dérivés de bis-hydroxyphénolbenzotriazole tels que ceux décrits dans les demandes US 5237071, US 5166355, GB-A-2303549, DE 19726184 et EP-A-893119 ; les dérivés de l'acide p-aminobenzoïque ; les polymères hydrocarbonés filtres et les silicones filtres tels que ceux décrits notamment dans la demande WO-93/04665.

Comme exemples de filtres organiques actifs dans l'UV-A et/ou l'UV-B, hydrophiles ou lipophiles, on peut citer :
l'acide p-aminobenzoïque,
le p-aminobenzoate oxyéthyléné (25mol),
le p-diméthylaminobenzoate de 2-éthylhexyle,
le p-aminobenzoate d'éthyle N-oxypropyléné
le p-aminobenzoate de glycérol,
le salicylate d'homomenthyle,
le salicylate de 2-éthylhexyle,
le salicylate de triéthanolamine,
le salicylate de 4-isopropylbenzyle,
le 4-ter-butyl-4'-méthoxy-dibenzoylméthane,
le 4-isopropyl-dibenzoylméthane,
le 4-méthoxy cinnamate de 2-éthylhexyle,
le diisopropyl cinnamate de méthyle,
le 4-méthoxy cinnamate d'isoamyle,
le 4-méthoxy cinnamate de diéthanolamine,
l'anthranilate de menthyle,
le 2-éthylhexyl-2-cyano-3,3'-diphénylacrylate,
l'éthyl-2-cyano-3,3'-diphénylacrylate,
l'acide 2-phényl benzimidazole 5-sulfonique et ses sels,
le 3-(4'-triméthylammonium)-benzylidèn-boman-2-on-méthylsulfate,
le 2-hydroxy-4-méthoxybenzophénone,
le 2-hydroxy-4-méthoxybenzophénone-5-sulfonate,
le 2,4-dihydroxybenzophénone,
le 2,2',4,4'-tétrahydroxybenzophénone,
le 2,2'-dihydroxy-4,4'diméthoxybenzophénone,
le 2-hydroxy-4-n-octoxybenzophénone,
le 2-hydroxy-4-méthoxy-4'-méthylbenzophénone,
l'acide α-(2-oxoborn-3-ylidène)-tolyl-4-sulfonique et ses sels solubles
le 3-(4'-sulfo)benzylidèn-bornan-2-one et ses sels solubles,
le 3-(4'méthylbenzylidène)-d,l-camphre,
le 3-benzylidène-d,l-camphre,
l'acide benzène 1,4-di(3-méthylidène-10-camphosulfonique) et ses sels solubles,
l'acide urocanique,
la 2,4,6-tris-[ p-(2'-éthylhexyl-1'-oxycarbonyl)anilino]-1,3,5-triazine,
la 2-[p-(tertiobutylamido)anilino]-4,6-bis-[(p-(2'-éthylhexyl-1'-oxycarbonyl)anilino]-1,3,5-triazine, .
la 2,4-bis {[4-2-éthyl-hexyloxy)]-2-hydroxy]-phenyl}-6-(4-méthoxy-phenyl)-1,3,5-triazine ;
le polymère de N-(2 et 4)-[(2-oxoborn-3-ylidèn)méthyl] benzyl]-acrylamide,
l'acide 1,4-bis-benzimidazolyl-phénylen-3,3',5,5'-tétrasulfonique et ses sels solubles.
le 2,2'-méthylène-bis-[6-(2H-benzotriazol-2-yl)-4-(1,1,3,3-tétraméthylbutyl)phénol],
le composé (2,2'-méthylène-bis-[6-(2H-benzotriazol-2-yl)-4-(methyl)phénol],
les polyorganosiloxanes à fonction benzalmalonate
les polyorganosiloxanes à fonction benzotriazole tel que le Drometrizole Trisiloxane.

Comme filtres organiques hydrophiles particulièrement utilisables dans la présente invention, on peut citer l'acide benzène 1,4-di(3-méthylidène-10-campho-sulfonique) vendue sous le nom commercial «MEXORYL SX» par CHIMEX et l'acide 2-phénylbenzimidazole-5-sulfonique vendu sous la dénomination commerciale «EUSOLEX 232» par la société MERCK.

Comme filtres organiques lipophiles particulièrement utilisables dans la présente invention, on peut citer :
- le 4-tert-butyl-4'-méthoxydibenzoylméthane vendu sous la dénomination commerciale «PARSOL 1789» par la société HOFFMANN LAROCHE ;
- l'octylméthoxycinnamate vendu sous la dénomination commerciale «PARSOL MCX» par la société HOFFMANN LAROCHE
- l'α-cyano- β,β -diphénylacrylate de 2-éthylhexyle (octocrylène) vendu sous la dénomination commerciale «UVINUL N 539» par la société BASF ;
- le 4-méthylbenzylidène camphre vendu sous la dénomination commerciale «EUSOLEX 6300» par MERCK ;
- la benzophénone-3 (oxybenzone) vendue sous la dénomination «UVINUL M40» par BASF;
- le salicylate de 2-éthylhexyle ou octylsalicylate vendue sous la dénomination commerciale NEO HELIOPAN OS par HAARMAAN & REIMER ;
- la 2,4,6-tris[p-(2'-éthylhexyl-1'-oxycarbonyl)anilino]-1,3,5-triazine vendue sous la dénomination «UVINUL T 150» par la Société BASF;
- le Drometrizole Trisiloxane vendu sous le nom commerciale «SILATRIZOLE» par la société RHODIA CHIMIE.

Une deuxième catégorie d'agents photoprotecteurs additionnels pouvant être utilisés dans les compositions selon l'invention est celle des pigments. De préférence, on met en oeuvre des nanopigments (taille moyenne des particules primaires : généralement entre 5 nm et 100 nm, de préférence entre 10 et 50 nm) minéraux d'oxydes métalliques enrobés ou non comme par exemple des nanopigments d'oxyde de titane (amorphe ou cristallisé sous forme rutile et/ou anatase), de fer, de zinc, de zirconium ou de cerium qui sont tous des agents photoprotecteurs bien connus en soi agissant par blocage physique (réflexion et/ou diffusion) du rayonnement UV. Des agents d'enrobage classiques sont par ailleurs l'alumine et/ou le stéarate d'aluminium ou encore les silicones. De tels nanopigments d'oxydes métalliques, enrobés ou non enrobés, sont en particulier décrits dans les demandes de brevets EP-A-0518772 et EP-A-0518773.

Le ou les (nano)pigments minéraux peuvent être présents dans les compositions selon l'invention à une teneur comprise entre 0,1% et 30%, de préférence de 0,5% à 10%, en poids, par rapport au poids total de la composition.

Les compositions selon l'invention peuvent également contenir des agents de bronzage et/ou de brunissage artificiels de la peau (agents autobronzants), tels que par exemple de la dihydroxyacétone (DHA).

Les compositions de l'invention peuvent comprendre en outre des adjuvants cosmétiques classiques notamment choisis parmi les corps gras, les solvants organiques, les épaississants, les adoucissants, les opacifiants, les stabilisants, les émollients, les agents anti-mousse, les agents hydratants, les parfums, les conservateurs, les polymères, les charges, les séquestrants, les bactéricides et/ou les absorbeurs d'odeur. les agents alcalinisants ou acidifiants, les tensio-actifs, les anti radicaux libres, les antioxydants, les vitamines comme les vitamines E et C, les α-hydroxyacides ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Bien entendu, l'homme de l'art veillera à choisir le ou les éventuels composés complémentaires cités ci-dessus et/ou leurs quantités de manière telle que les propriétés avantageuses attachées intrinsèquement à l'association [système photoprotecteur + mélange de polyéthylènes] conforme à l'invention ne soit pas, ou substantiellement pas, altérée par la ou les adjonctions envisagées.

Les compositions concernées par l'invention peuvent être préparées selon les techniques bien connues de l'homme de l'art, destinées à la préparation des émulsions de type huile-dans-eau.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, par rapport à l'ensemble de la formulation.

Des exemples concrets, mais nullement limitatifs, illustrant l'invention, vont maintenant être donnés.

Dans tous les exemples qui suivent, les quantités sont exprimées en % de poids par rapport au poids total de la composition.

### EXEMPLE 1 :

On a préparé une formulation antisolaire A selon l'invention se présentant sous la forme d'une émulsion de type huile-dans-eau et contenant:

| **Formulation A** | **Quantité % en poids** |
|---|---|
| Octocrylène (UVINUL N539) | **10** |
| Octyl salicylate (NEO HELIOPAN OS) | **5** |
| Benzophénone-3 (UVINUL M 40) | **6** |
| 4-tertio-butyl 4'-méthoxy dibenzoylméthane (PARSOL 1789) | **3** |
| Cyclométhicone | **7** |
| Glycols | **8** |
| Isopropyl palmitate | **2,5** |
| Copolymère PVP/Eicosène | **1** |
| Acide stéarique | **1** |
| Diméthicone | **1** |
| Mélange stéarate de glycéryle/stéarate PEG-100 (ARLACEL 165) | **1** |
| Copolymère réticulé acrylates/ acrylate d'alkyle en C₁₀₋₃₀ (PEMULEN TR1) | **0,2** |
| Mélange polyéthylène à fonction acide carboxylique terminale/polyéthylène de poids moléculaire 350, d'indice d'acide 115 et de point de fusion 89°C (PERFORMACID 350) | **2** |
| Gomme de Xanthane | **0,1** |
| Triéthanolamine | **qs** |
| Conservateurs | **qs** |
| Eau | **qsp 100** |

On a ensuite préparé une formulation antisolaire A' comparative, de même support que formulation A mais ne contenant pas de mélange polyéthylène à fonction acide carboxylique terminale/polyéthylène.

Pour chacune des compositions A et A', on a ensuite déterminé le facteur de protection solaire (SPF) qui lui était attaché. Celui-ci a été déterminé en utilisant la méthode *in vitro* décrite par B.L. DIFFEY et al. dans J. Soc. Cosmet. Chem. 40-127-133 (1989) ; cette méthode consiste à déterminer les facteurs de protection monochromatiques tous les 5 nm dans une gamme de longueurs d'onde de 290 à 400 nm et à calculer à partir de ceux-ci le facteur de protection solaire selon une équation mathématique donnée.

Les résultats (valeur moyenne correspondant à cinq essais) sont regroupés dans le tableau (I) ci-dessous :

**Tableau (I):**

| Composition | A' (comparative) sans mélange de polyéthylènes | A (invention) avec mélange de polyéthylènes |
|---|---|---|
| SPF moyen (écart type) | 19.8 (5.1) | 31,5 (4,9) |

Ces résultats montrent clairement que l'ajout dans un support émulsion huile/eau d'un mélange polyéthylène à fonction acide carboxylique terminale/polyéthylène à un système photoprotecteur constitué d'octocrylène, d'octylsalicylate, de 4-tertio-butyl 4'-méthoxy dibenzoylméthane, de benzophénone-3 permet d'augmenter significativement son pouvoir photoprotecteur.

### EXEMPLE 2 :

On a préparé une formulation antisolaire B selon l'invention se présentant sous la forme d'une émulsion de type huile-dans-eau et contenant :

| **Formulation B** | **Quantité % en poids** |
|---|---|
| Octocrylène (UVINUL N539) | **10** |
| Octyl salicylate (NEO HELIOPAN OS) | **5** |
| Benzophénone-3 (UVINUL M 40) | **6** |
| 4-tertio-butyl 4'-méthoxy dibenzoylméthane (PARSOL 1789) | **3** |
| Glycols | **8** |
| Benzoate d'alkyle en C₁₂-C₁₅ (FINSOLV TN) | **2,5** |
| Copolymère PVP/Eicosène | **1** |
| Acide stéarique | **1** |
| Mélange stéarate de glycéryle/stéarate PEG-100 (ARLACEL 165) | **1** |
| Copolymère réticulé acrylates/ acrylate d'alkyle en C₁₀₋₃₀ (PEMULEN TR1) | **0,2** |
| Mélange polyéthylène à fonction acide carboxylique terminale/polyéthylène de poids moléculaire 350, d'indice d'acide 115 et de point de fusion 89°C (PERFORMACID 350) | **0,5** |
| Gomme de Xanthane | **0,1** |
| Triéthanolamine | **qs** |
| Conservateurs | **qs** |
| Eau | **qsp 100** |

On a ensuite préparé une formulation antisolaire B' selon l'invention, de même support que la formulation B mais contenant 2% en poids mélange polyéthylène à fonction acide carboxylique terminale/polyéthylène.

Pour chacune des compositions B et B', on a ensuite déterminé le facteur de protection solaire (SPF) selon la même méthode que l'exemple 1.

Les résultats (valeur moyenne correspondant à cinq essais) sont regroupés dans le tableau (II) ci-dessous :

**Tableau (II) :**

| Composition | B (invention) avec 0,5 % de mélange de polyéthylènes | B' (invention) Avec 2% de mélange de polyéthylènes |
|---|---|---|
| SPF moyen (écart type) | 31,3 (7,7) | 38,7 (6.5) |

Ces résultats montrent clairement que l'ajout dans un support émulsion huile/eau d'un mélange polyéthylène à fonction acide carboxylique terminale/polyéthylène à un système photoprotecteur constitué d'octocrylène, d'octylsalicylate, de 4-tertio-butyl 4'-méthoxy dibenzoylméthane, de benzophénone-3 permet d'augmenter significativement son pouvoir photoprotecteur.

### EXEMPLE 3 :

On a préparé une formulation antisolaire C selon l'invention se présentant sous la forme d'une émulsion de type huile-dans-eau et contenant:

| **Formulation C** | **Quantité % en poids** |
|---|---|
| Octocrylène (UVINUL N539) | **10** |
| 4-méthylbenzylidène camphre (EUSOLEX 6300) | **4** |
| Acide 2-phénylbenzimidazole-5-sulfonique (EUSOLEX 232) | **1,5** |
| Acide benzène 1,4-di(3-méthylidène-10-campho-sulfonique) (MEXORYL SX) | **1** |
| 4-tertio-butyl 4'-méthoxy dibenzoylméthane (PARSOL 1789) | **3** |
| Cyclométhicone | **7** |
| Diméthicone | **1** |
| Glycols | **8** |
| Isopropyl palmitate | **2,5** |
| Copolymère PVP/Eicosène | **1** |
| Acide stéarique | **1** |
| Mélange stéarate de glycéryle/stéarate PEG-100 (ARLACEL 165) | **1,5** |
| Copolymère réticulé acrylates/ acrylate d'alkyle en C₁₀₋₃₀ (PEMULEN TR1) | **0,23** |
| Mélange polyéthylène à fonction acide carboxylique terminale/polyéthylène de poids moléculaire 350, d'indice d'acide 115 et de point de fusion 89°C (PERFORMACID 350) | **1** |
| Gomme de Xanthane | **0,1** |
| Triéthanolamine | **qs** |
| Conservateurs | **qs** |
| Eau | **qsp 100** |

On a ensuite préparé une formulation antisolaire C' selon l'invention, de même support que la formulation C mais contenant 2% en poids mélange polyéthylène à fonction acide carboxylique terminale/polyéthylène.

On a ensuite préparé une formulation antisolaire C" comparative, de même support que la formulation C mais ne contenant pas de mélange polyéthylène à fonction acide carboxylique terminale/polyéthylène.

Pour chacune des compositions C, C et C", on a ensuite déterminé le facteur de protection solaire (SPF) selon la même méthode que l'exemple 1. Les résultats (valeur moyenne correspondant à cinq essais) sont regroupés dans le tableau (III) ci-dessous :

**Tableau (III) :**

| Composition | C" (comparative) sans mélange de polyéthylènes | C (invention) avec 1 % de mélange de polyéthylènes | C' (invention) avec 2% de mélange de polyéthylènes |
|---|---|---|---|
| SPF moyen (écart type) | 13,2 (2,8) | 34,6 (5,2) | 42,1 (6.7) |

Ces résultats montrent clairement que l'ajout dans un support émulsion huile/eau d'un mélange polyéthylène à fonction acide carboxylique terminale/polyéthylène à un système photoprotecteur constitué d'octocrylène, de 4-méthylbenzylidène camphre, de 4-tertio-butyl 4'-méthoxy dibenzoylméthane, d'acide benzène 1,4-di(3-méthylidène-10-campho-sulfonique) et d'acide 2-phénylbenzimidazole-5-sulfonique permet d'augmenter significativement son pouvoir photoprotecteur.

### EXEMPLE 4 :

On a préparé une formulation antisolaire D selon l'invention se présentant sous la forme d'une émulsion de type huile-dans-eau et contenant:

| **Formulation D** | **Quantité % en poids** |
|---|---|
| Octocrylène (UVINUL N539) | **10** |
| Octyl salicylate (NEO HELIOPAN OS) | **5** |
| Benzophénone-3 (UVINUL M 40) | **6** |
| 4-tertio-butyl 4'-méthoxy dibenzoylméthane (PARSOL 1789) | **3** |
| Cyclométhicone | **7** |
| Glycols | **8** |
| Nanopigments TiO₂ (MT100T) | **3** |
| Isopropyl palmitate | **2,5** |
| Copolymère PVP/Eicosène | **1** |
| Acide stéarique | **1** |
| Diméthicone | **1** |
| Mélange stéarate de glycéryle/stéarate PEG-100 (ARLACEL 165) | **1** |
| Copolymère réticulé acrylates/ acrylate d'alkyle en C10-30 (PEMULEN TR1) | **0,2** |
| Mélange polyéthylène à fonction acide carboxylique terminale/polyéthylène de poids moléculaire 350, d'indice d'acide 115 et de point de fusion 89°C (PERFORMACID 350) | **2** |
| Gomme de Xanthane | **0,1** |
| Triéthanolamine | **qs** |
| Conservateurs | **qs** |
| Eau | **qsp 100** |

On a ensuite préparé une formulation antisolaire D' comparative, de même support que la formulation D mais ne contenant pas de mélange polyéthylène à fonction acide carboxylique terminale/polyéthylène. Pour chacune des compositions D et D', on a ensuite déterminé le facteur de protection solaire (SPF) selon la même méthode que l'exemple 1.

Les résultats (valeur moyenne correspondant à cinq essais) sont regroupés dans le tableau (IV) ci-dessous :

**Tableau (IV) :**

| Composition | D (invention) avec 2 % de mélange de polyéthylènes | D' (comparative) sans mélange de polyéthylènes |
|---|---|---|
| SPF moyen (écart type) | 48,0 (11,2) | 80,5 (12,2) |

Ces résultats montrent clairement que l'ajout d'un mélange polyéthylène à fonction acide carboxylique terminale/polyéthylène à un système photoprotecteur constitué d'octocrylènè, d'octylsalicylate, de 4-tertio-butyl 4'-méthoxy dibenzoylméthane, de benzophénone-3 et de TiO₂ permet d'augmenter significativement son pouvoir photoprotecteur.

### EXEMPLE 5 :

On a préparé une formulation antisolaire E selon l'invention se présentant sous la forme d'une émulsion de type huile-dans-eau et contenant:

| **Formulation E** | **Quantité % en poids** |
|---|---|
| Octocrylène (UVINUL N539) | **10** |
| 4-tertio-butyl 4'-méthoxy dibenzoylméthane (PARSOL 1789) | **2** |
| Acide benzène 1,4-di(3-méthylidène-10-campho-sulfonique) (MEXORYL SX) | **2** |
| Cyclométhicone | **7** |
| Glycols | **15** |
| Isopropyl palmitate | **2,2** |
| Copolymère PVP/Eicosène | **1** |
| Acide stéarique | **1,5** |
| Alcool stéarylique | **0,8** |
| Diméthicone | **1** |
| Mélange stéarate de glycéryle/stéarate PEG-100 (ARLACEL 165) | **1,5** |
| Copolymère réticulé acrylates/ acrylate d'alkyle en C10-30 (PEMULEN TR1) | **0,2** |
| Hydroxypropylméthylcellulose | **0,1** |
| Polymère d'acide acrylique réticulé (CARBOMER) | **0,1** |
| Mélange polyéthylène à fonction acide carboxylique terminale/polyéthylène de poids moléculaire 550, d'indice d'acide 72,5 et de point de fusion 101°C (PERFORMACID 550) | **4** |
| Gomme de Xanthane | **0,1** |
| Triéthanolamine | **qs** |
| Conservateurs | **qs** |
| Eau | **qsp 100** |

La composition présente un SPF in vitro 13,9 ± 1,6.

## Revendications

1. Composition cosmétique et/ou dermatologique, destinée à la protection de la peau et/ou des cheveux contre le rayonnement ultraviolet, **caractérisée par le fait qu'**elle comprend, dans un support cosmétiquement acceptable du type émulsion huile-dans-eau, au moins :
(a) un système photoprotecteur capable de filtrer le rayonnement UV ;
(b) un mélange de polyéthylènes constitué d'au moins un polyéthylène à fonction acide carboxylique terminale sous forme libre, partiellement ou totalement neutralisée et d'au moins un polyéthylène ; ledit mélange ayant un poids moléculaire moyen en nombre allant de 300 à 800, présente un indice d'acide allant de 60 à 120 et un point de fusion allant de 80 à 120°C.

2. Composition selon la revendication 1, selon laquelle le mélange de polyéthylène comprend de 60 à 99% en polyéthylène à fonction acide carboxylique terminale et de 1 à 40% en poids de polyéthylène par rapport poids total du mélange.

3. Composition selon la revendication 2, selon laquelle le mélange de polyéthylène comprend de 80 à 90 % en poids en polyéthylène à fonction acide carboxylique terminale et de 10 à 20% en poids de polyéthylène par rapport poids total du mélange.

4. Composition selon l'une quelconque des revendications 1 à 3, où la fonction acide carboxylique terminale peut être éventuellement partiellement ou totalement neutralisée.

5. Composition selon l'une quelconque des revendications 1 à **4**, où le mélange de polyéthylènes est choisi dans le groupe constitué par :
- le mélange polyéthylène à fonction acide carboxylique terminale/polyéthylène de poids moléculaire 350, d'indice d'acide 115 et de point de fusion 89°C ;
- le mélange polyéthylène à fonction acide carboxylique terminale/polyéthylène de poids moléculaire 460, d'indice d'acide 94 et de point de fusion 94°C ;
- le mélange polyéthylène à fonction acide carboxylique terminale/polyéthylène de poids moléculaire 550, d'indice d'acide 72,5 et de point de fusion 101°C ;
- le mélange polyéthylène à fonction acide carboxylique terminale/polyéthylène de poids moléculaire 700, d'indice d'acide 63 et de point de fusion 110°C.

6. Composition selon l'une quelconque des revendications 1 à 5, où le mélange de polyéthylènes est présent à des concentrations allant de préférence de 0,1 à 10% en poids par rapport au poids total de la composition et plus particulièrement de 0,2 à 5% en poids.

7. Composition selon l'une quelconque des revendications 1 à 6, où le système photoprotecteur est présent à une teneur allant de 0,1 % à 30 % en poids et de préférence de 0,5 à 15 %, en poids, par rapport au poids total de la composition.

8. Composition selon l'une quelconque des revendications 1 à 7, où le système photoprotecteur est constitué par un ou plusieurs filtres organiques hydrophiles et/ou un ou plusieurs filtres organiques lipophiles et/ou un ou plusieurs (nano)pigments minéraux actifs dans l'UV-A et/ou l'UV-B.

9. Composition selon la revendication 8, où les filtres organiques actifs dans l'UV-A et/ou l'UV-B sont choisis parmi les dérivés cinnamiques ; les dérivés de dibenzoylméthane ; les dérivés salicyliques, les dérivés du camphre ; les dérivés de triazine ; les dérivés de la benzophénone ; les dérivés de β,β'-diphénylacrylate ; les dérivés de benzimidazole ; les dérivés bis-benzoazolyle ; les dérivés de bis-hydroxyphénolbenzotriazole ; les dérivés de l'acide p-aminobenzoïque ; les polymères hydrocarbonés filtres et les silicones filtres.

10. Composition selon la revendication 9 où les filtres organiques hydrophiles actifs dans l'UV-A et/ou l'UV-B sont choisis dans le groupe constitué par l'acide benzène 1,4-di(3-méthylidène-10-campho-sulfonique) et l'acide 2-phénylbenzimidazole-5-sulfonique.

11. Composition selon la revendication 9 où les filtres organiques lipophiles actifs dans l'UV-A et/ou l'UV-B sont choisis sont choisis dans le groupe constitué par
- le 4-tert-butyl-4'-méthoxydibenzoylméthane;
- l'octylméthoxycinnamate ;
- l'α-cyano-β,β-diphénylacrylate de 2-éthylhexyle (octocrylène) ;
- le 4-méthylbenzylidène camphre;
- la benzophénone-3 (oxybenzone) ;
- le salicylate de 2-éthylhexyle ou octylsalicylate ;
- la 2,4,6-tris[p-(2'-éthylhexyl-1'-oxycarbonyl)anilino]-1,3,5-triazine ;
- le Drométrizole Trisiloxane .

12. Composition selon la revendication 8, **caractérisée par le fait que** le système photoprotecteur comprend au moins des pigments ou des nanopigments d'oxydes métalliques, enrobés ou non, capables de bloquer physiquement, par diffusion et/ou réflection, le rayonnement UV.

13. Composition selon la revendication 12, **caractérisée par le fait que** lesdits pigments ou nanopigments sont choisis parmi les oxydes de titane, de zinc, de fer, de zirconium, de cérium et leurs mélanges, enrobés ou non.

14. Composition selon l'une quelconque des revendications 1 à 13, **caractérisée par le fait qu'**elles comprend en outre au moins un agent de bronzage et/ou de brunissage artificiel de la peau.

15. Composition selon l'une quelconque des revendications 1 à 14, **caractérisée par le fait qu'**elles comprend en outre au moins un additif choisi dans le groupe constitué par les corps gras, les solvants organiques, les épaississants, les adoucissants, les opacifiants, les stabilisants, les émollients, les agents anti-mousse, les agents hydratants, les parfums, les conservateurs, les polymères, les charges, les séquestrants, les bactéricides et/ou les absorbeurs d'odeur. les agents alcalinisants ou acidifiants, les tensio-actifs, les anti radicaux libres, les antioxydants, les vitamines, les α-hydroxyacides.

16. Utilisation d'un mélange de polyéthylènes constitué d'au moins un polyéthylène à fonction acide carboxylique terminale et d'au moins un polyéthylène tel que défini dans l'une quelconque des revendication 1 à 4, pour la fabrication d'une composition destinée à la protection de la peau et/ou des cheveux contre le rayonnement ultraviolet, comprenant dans un support du type émulsion huile dans eau au moins un système photoprotecteur capable de filtrer le rayonnement UV, comme agent permettant d'augmenter le facteur de protection solaire.

## Patentansprüche

1. Kosmetische und/oder dermatologische Zusammensetzung, die für den Schutz der Haut und/oder der Haare gegen W-Strahlung vorgesehen ist, **dadurch gekennzeichnet, dass** sie in einem kosmetisch akzeptablen Träger vom Typ einer Öl-in-Wasser-Emulsion zumindest enthält:
(a) ein Lichtschutzsystem, das die UV-Strahlung filtern kann; und
(b) ein Gemisch aus Polyethylenen, das aus mindestens einem Polyethylen mit endständiger Carboxyfunktion in freier Form, die ganz oder teilweise neutralisiert ist, und mindestens einem Polyethylen besteht; wobei das Gemisch eine zahlenmittlere Molmasse von 300 bis 800 aufweist, einen Säureindex von 60 bis 120 besitzt und einen Schmelzpunkt von 80 bis 120 °C hat.

2. Zusammensetzung nach Anspruch 1, wobei das Polyethylengemisch 60 bis 99 Gew.-% Polyethylen mit endständiger Carboxyfunktion und 1 bis 40 Gew.-% Polyethylen, bezogen auf das Gesamtgewicht des Gemisches, enthält.

3. Zusammensetzung nach Anspruch 2, wobei das Polyethylengemisch 80 bis 90 Gew.-% Polyethylen mit endständiger Carboxyfunktion und 10 bis 20 Gew.-% Polyethylen, bezogen auf das Gesamtgewicht des Gemisches, enthält.

4. Zusammensetzung nach einem der Ansprüche 1 bis 3, wobei die endständige Carboxyfunktion ganz oder teilweise neutralisiert sein kann.

5. Zusammensetzung nach einem der Ansprüche 1 bis 4, wobei das Gemisch von Polyethylenen ausgewählt ist unter:
- dem Gemisch Polyethylen mit endständiger Carboxyfunktion/Polyethylen mit einer Molmasse von 350, einem Säureindex von 115 und einem Schmelzpunkt von 89 °C;
- dem Gemisch Polyethylen mit endständiger Carboxyfunktion/Polyethylen mit einer Molmasse von 460, einem Säureindex von 94 und einem Schmelzpunkt von 94 °C;
- dem Gemisch Polyethylen mit endständiger Carboxyfunktion/Polyethylen mit einer Molmasse von 550, einem Säureindex von 72,5 und einem Schmelzpunkt von 101 °C; und
- dem Gemisch Polyethylen mit endständiger Carboxyfunktion/Polyethylen mit einer Molmasse von 700, einem Säureindex von 63 und einem Schmelzpunkt von 110 °C.

6. Zusammensetzung nach einem der Ansprüche 1 bis 5, wobei das Gemisch von Polyethylenen in Konzentrationen von vorzugsweise 0,1 bis 10 Gew.-%, bezogen auf das Gesamtgewicht der Zusammensetzung, und insbesondere 0,2 bis 5 Gew.-% enthalten ist.

7. Zusammensetzung nach einem der Ansprüche 1 bis 6, wobei das Lichtschutzsystem in einer Menge von 0,1 bis 30 Gew.-% und vorzugsweise 0,5 bis 15 Gew.-%, bezogen auf das Gesamtgewicht der Zusammensetzung, vorliegt.

8. Zusammensetzung nach einem der Ansprüche 1 bis 7, wobei das Lichtschutzsystem aus einem oder mehreren hydrophilen organischen Filtern und/oder einem oder mehreren lipophilen organischen Filtern und/ oder einem oder mehreren anorganischen (Nano)pigmenten, die im UV-A- und/ oder UV-B-Bereich wirksam sind, besteht.

9. Zusammensetzung nach Anspruch 8, wobei die im UV-Aund/oder UV-B-Bereich wirksamen organischen Filter unter den Zimtsäurederivaten; Dibenzoylmethanderivaten; Salicylsäurederivaten, Campherderivaten; Triazinderivaten; Benzophenonderivaten; β,β'-Diphenylaerylatderivaten; Benzimidazolderivaten; Bisbenzoazolylderivaten; Bis-hydroxyphenolbenzotriazolderivaten; p-Aminobenzoesäurederivaten; Filterpolymeren auf Kohlenwasserstoffbasis und Siliconfiltern ausgewählt sind.

10. Zusammensetzung nach Anspruch 9, wobei die hydrophilen organischen Filter, die im UV-A- und/oder UV-B-Bereich wirksam sind, unter 1,4-Di(3-methyliden-10-camphersulfonsäure) und 2-Phenylbenzimidazol-5-sulfonsäure ausgewählt sind.

11. Zusammensetzung nach Anspruch 9, wobei die im UV-Aund/oder UV-B-Bereich wirksamen, lipophilen organischen Filter ausgewählt sind unter:
- 4-*t*-Butyl-4'-methoxydibenzoylmethan;
- Octylmethoxycinnamat;
- 2-Ethylhexyl-α-cyano-β,β'-diphenylacrylat (Octocrylen);
- 4-Methylbenzylidencampher;
- Benzophenon-3 (Oxybenzon);
- 2-Ethylhexylsalicylat oder Octylsalicylat;
- 2,4,6-Tris[p-(2'-ethylhexyl-1'-oxycarbonyl)anilino]-1,3,5-triazin; und
- Drometrizole Trisiloxane.

12. Zusammensetzung nach Anspruch 8, **dadurch gekennzeichnet, dass** das Lichtschutzsystem zumindest Pigmente oder Nanopigmente von Metalloxiden enthält, die umhüllt oder nicht umhüllt sind und befähigt sind, die UV-Strahlung physikalisch durch Streuung und/ oder Reflexion zu sperren.

13. Zusammensetzung nach Anspruch 12, **dadurch gekennzeichnet, dass** die Pigmente oder Nanopigmente unter den umhüllten oder nicht umhüllten Oxiden von Titan, Zink, Eisen, Zirkonium oder Cer und deren Gemischen ausgewählt sind.

14. Zusammensetzung nach einem der Ansprüche 1 bis 13, **dadurch gekennzeichnet, dass** sie ferner mindestens ein Mittel zum Braunfärben und/oder zur künstlichen Bräunung der Haut enthält.

15. Zusammensetzung nach einem der Ansprüche 1 bis 14, **dadurch gekennzeichnet, dass** sie ferner mindestens einen Zusatzstoff enthält, der unter den Fettsubstanzen, organischen Lösungsmitteln, Verdickungsmitteln, beruhigenden Stoffen, Trübungsmitteln, Stabilisatoren, Emollientien, Schaumverhütungsmitteln, Hydratisierungsmitteln, Parfums, Konservierungsmitteln, Polymeren, Füllstoffen, Maskierungsmitteln, Bakteriziden und/oder Geruchsabsorbern, Alkalisierungsmitteln oder Ansäuerungsmitteln, grenzflächenaktiven Stoffen, Radikalfängern für freie Radikale, Antioxidantien, Vitaminen und α-Hydroxysäuren ausgewählt ist.

16. Verwendung eines Gemisches von Polyethylenen, das aus mindestens einem Polyethylen mit endständiger Carboxyfunktion und mindestens einem Polyethylen besteht, wie es in einem der Ansprüche 1 bis 4 definiert wurde, zur Herstellung einer Zusammensetzung, die für den Schutz der Haut und/oder der Haare gegen UV-Strahlung vorgesehen ist und die in einem Träger vom Typ einer Öl-in-Wasser-Emulsion mindestens ein Lichtschutzsystem enthält, das die UV-Strahlung filtern kann, als Mittel zur Erhöhung des Lichtschutzfaktors.

## Claims

1. Cosmetic and/or dermatological composition intended for protecting the skin and/or the hair against ultraviolet radiation, **characterized in that** it comprises, in a cosmetically acceptable support of the oil-in-water emulsion type, at least:
(a) one sunscreen system capable of screening out UV radiation;
(b) one mixture of polyethylenes consisting of at least one polyethylene containing a carboxylic acid end function in free, partially neutralized or totally neutralized form and at least one polyethylene, the said mixture having a number-average molecular weight ranging from 300 to 800, an acid number ranging from 60 to 120 and a melting point ranging from 80°C to 120°C.

2. Composition according to Claim 1, in which the polyethylene mixture comprises from 60% to 99% by weight of polyethylene containing a carboxylic acid end function and from 1% to 40% by weight of polyethylene relative to the total weight of the mixture.

3. Composition according to Claim 2, in which the polyethylene mixture comprises from 80% to 90% by weight of polyethylene containing a carboxylic acid end function and from 10% to 20% by weight of polyethylene relative to the total weight of the mixture.

4. Composition according to any one of Claims 1 to 3, in which the carboxylic acid end function may .optionally be partially neutralized or totally neutralized.

5. Composition according to any one of Claims 1 to 4, in which the polyethylene mixture is chosen from the group consisting of:
- the mixture of polyethylene containing a carboxylic acid end function/polyethylene of molecular weight 350, of acid number 115 and of melting point 89°C;
- the mixture of polyethylene containing a carboxylic acid end function/polyethylene of molecular weight 460, of acid number 94 and of melting point 94°C;
- the mixture of polyethylene containing a carboxylic acid end function/polyethylene of molecular weight 550, of acid number 72.5 and of melting point 101°C;
- the mixture of polyethylene containing a carboxylic acid end function/polyethylene of molecular weight 700, of acid number 63 and of melting point 110°C.

6. Composition according to any one of Claims 1 to 5, in which the polyethylene mixture is present at concentrations preferably ranging from 0.1% to 10% by weight relative to the total weight of the composition and more particularly from 0.2% to 5% by weight.

7. Composition according to any one of Claims 1 to 6, in which the sunscreen system is present at a content ranging from 0.1% to 30% by weight and preferably from 0.5% to 15% by weight relative to the total weight of the composition.

8. Composition according to any one of Claims 1 to 7, in which the sunscreen system consists of one or more hydrophilic organic screening agents and/or one or more lipophilic organic screening agents and/or one or more mineral (nano)pigments that are active in the UV-A and/or UV-B range.

9. Composition according to Claim 8, in which the UV-A-active and/or UV-B-active organic screening agents are chosen from cinnamic derivatives; dibenzoylmethane derivatives; salicylic derivatives; camphor derivatives; triazine derivatives; benzophenone derivatives; β,β'-diphenylacrylate derivatives; benzimidazole derivatives; bis-benzazolyl derivatives; bis-hydroxyphenolbenzotriazole derivatives; p-aminobenzoic acid derivatives; screening hydrocarbon-based polymers and screening silicones.

10. Composition according to Claim 9, in which the UV-A-active and/or UV-B-active hydrophilic organic screening agents are chosen from the group consisting of benzene-1,4-bis(3-methylidene-10-camphor-sulphonic acid) and 2-phenylbenzimidazole-5-sulphonic acid.

11. Composition according to Claim 9, in which the UV-A-active and/or UV-B-active lipophilic organic screening agents are chosen from the group consisting of
- 4-tert-butyl-4'-methoxydibenzoylmethane;
- octyl methoxycinnamate;
- 2-ethylhexyl α-cyano-β,β-diphenylacrylate (octocrylene);
- 4-methylbenzylidenecamphor;
- benzophenone-3 (oxybenzone);
- 2-ethylhexyl salicylate or octyl salicylate;
- 2,4,6-tris[p-(2'-ethylhexyl-1'-oxycarbonyl)-anilino]-1,3,5-triazine;
- drometrizole trisiloxane.

12. Composition according to Claim 8, **characterized in that** the sunscreen system comprises at least coated or uncoated metal oxide pigments or nanopigments capable of physically blocking out UV radiation by scattering and/or reflection.

13. Composition according to Claim 12, **characterized in that** the said pigments or nanopigments are chosen from titanium oxide, zinc oxide, iron oxide, zirconium oxide and cerium oxide and mixtures thereof, which may be coated or uncoated.

14. Composition according to any one of Claims 1 to 13, **characterized in that** it also comprises at least one agent for artificially tanning and/or bronzing the skin.

15. Composition according to any one of Claims 1 to 14, **characterized in that** it also comprises at least one additive chosen from the group consisting of fatty substances, organic solvents, thickeners, softeners, opacifiers, stabilizers, emollients, antifoams, moisturizers, fragrances, preserving agents, polymers, fillers, sequestering agents, bactericides and/or odour absorbers, acidifying or basifying agents, surfactants, free-radical scavengers, antioxidants, vitamins and α-hydroxy acids.

16. Use of a mixture of polyethylenes consisting of at least one polyethylene containing a carboxylic acid end function and at least one polyethylene as defined in any one of Claims 1 to 4, for the manufacture of a composition intended for protecting the skin and/or the hair against ultraviolet radiation, comprising, in a support of the oil-in-water emulsion type, at least one sunscreen system capable of screening out UV radiation, as an agent for increasing the sun protection factor.
